# EUROPEAN PATENT APPLICATION

(11) **EP 3 456 735 A1**
(43) Date of publication of application: **20.03.2019**
(21) Application number: 18202044.6
(22) Date of filing: 26.06.2013
(51) Int. Cl.: C07K 16/08, C07K 16/28

(54) **BINDING MOLECULES TARGETING PATHOGENS**

(30) Priority: 26.06.2012 US 201261664475 P; 03.07.2012 US 201261667859 P
(62) Divisional of application: 13737682.8
(71) Applicant: Apo-T B.V., 3818 EE Amersfoort (NL)
(72) Inventor: Renes, Johan, 3818 LW Amersfoort (NL); Steverink, Paulus Johannes Gerardus Maria, 1272 GB Huizen (NL); Willemsen, Ralph Alexander, deceased (NL)
(74) Representative: Nederlandsch Octrooibureau

(57) **Abstract**

A first aspect of the invention relates to the field of binding molecules targeted at pathogens. The present invention further relates to proteinaceous binding molecules targeting cells displaying pathogen-associated molecular patterns, in particular targeting cell surface molecules associated with or derived from pathogens, more in particular cell surface proteins displaying peptides from intracellular (pathogen associated) proteins.

## Description

### Technical field

The invention relates to the field of binding molecules targeted at pathogens. The invention also relates to proteinaceous binding molecules targeting cells displaying pathogen-associated molecular patterns, in particular targeting cell surface molecules associated with or derived from pathogens, more in particular cell surface proteins displaying peptides from intracellular (pathogen asociated) proteins.

### Background

The infectious disease community is continuously searching for new or improved molecules that are efficacious in aiding the containment or the eradication of pathogens associated with the animal or human body. A shortcoming of nowadays therapeutic approaches is the insufficient closure of the body's gates through which pathogens manage to escape the defense mechanisms offered jointly by therapeutic molecules and the body's immune system. The efficacy of therapeutic molecules targeting a binding site on a pathogen or on an infected cell is severely challenged due to high mutation rates of the pathogen surface molecules. Thus, a binding molecule specific for a single antigen may lose its therapeutic benefits once this target binding site is mutated in a way that affinity of the binding molecule is efficiently lowered or even completely abolished. In fact, the ability to escape the animal or human body's immune system and to circumvent the therapeutic benefits of binding molecules, by mutations and/or by hiding inside cells, is a key determinant in the virulence of pathogens. So, improved therapeutic options are therefore intensively sought.

### Embodiments of the invention

The present invention provides proteinaceous binding molecules with improved specificity for pathogens affecting the animal or human body. In one embodiment this is achieved by targeting (at least two different) at least four the same or different binding sites on a pathogen or on an infected cell with the multivalent proteinaceous molecules of the invention, binding to at least one or to several binding sites still remains when one or several of the other initially targeted binding sites is not available (any more). Thereby, the chance of (immune) escape is sufficiently reduced. In this way, at least part of the desired therapeutic effect is maintained. To accomplish this, the present invention provides proteinaceous molecules comprising binding domains that bind at least four the same or different binding sites on pathogens or on infected cells or on aberrant cells altered upon infection. These proteinaceous molecules of the invention with improved therapeutic efficacy provide a solution to a number of current technical problems, which solution is further described by the embodiments below and provided in the claims. In another embodiment the present invention provides molecules that induce apoptosis in cells infected by pathogens by binding to at least four cell surface associated proteins associated with or derived from a pathogen.

Thus the invention provides a proteinaceous molecule comprising at least four the same and/or different (different) specific binding domains for (different) binding sites wherein said proteinaceous molecule comprises a single polypeptide chain.

In one embodiment of the invention the (at least two different) binding sites targeted by proteinaceous molecules of the invention are present on the surface of pathogens. In a further embodiment of the invention the binding sites targeted by proteinaceous molecules of the invention are present on the surface of cells infected by pathogens. In yet another embodiment of the invention, the binding sites targeted by proteinaceous molecules of the invention are presented by MHC molecules on the surface of cells in the body that present pathogen epitopes upon exposure of the body to pathogens. In the most preferred embodiment of the invention, the different binding sites targeted by proteinaceous molecules of the invention are at least preferentially, preferably uniquely present on the targeted pathogen or targeted (infected) cell. In yet another embodiment of the invention, at least one of the targeted binding sites is uniquely present on the targeted pathogen or cell. In one preferred embodiment of the invention the at least four binding sites targeted by proteinaceous molecules of the invention are pathogen derived peptides presented at the surface of pathogen-invaded cells in the context of MHC-1 and/or MHC-2. These latter molecules of the invention, when bound to the infected cell with all at least four binding domains, will induce apoptosis of the infected cell.

According to the invention *proteinaceous molecules* are molecules comprising at least a string of amino acid residues that can be obtained as an expression product from a single messenger RNA molecule. These single chain proteinaceous molecules may associate with further proteinaceous molecules, in particular associations that occur in nature. In addition, according to the invention the proteinaceous molecules may comprise carbohydrates such as N-linked and O-linked glycosylations, disulphide bonds, phosphorylations, sulphatations, etc., as a result of any post-translational modification, and/or any other modification such as those resulting from chemical modifications (e.g. linking of effector moieties). In one embodiment of the invention the proteinaceous molecules comprise a single polypeptide chain comprising at least two, preferably at least four specific binding domains. In a preferred embodiment, the proteinaceous molecules of the invention comprise binding domains separated by at least one linker, preferably at least three linkers. Of course, the proteinaceous molecules of the invention can also comprise other functionalities, for example provided with protein domains or amino acid sequences, linked through peptide bonds or through any linker chemistry known in the art. Proteinaceous molecules of the invention that recognize pathogen derived peptides in the context of MHC-1 or MHC-2 further encompass immunoglobulins. Immunoglobulins of the invention are preferably antibodies, but fragments and/or derivatives such as Fab and/or ScFv can also be used. Even more preferred immunoglobulins of the invention are antibodies of the immunoglobulin G (IgG) type. These antibodies may be provided with cytotoxic agents (so called antibody drug conjugates (ADC)).

A *polypeptide chain* is defined as a string of amino acid residues. *Specific binding domains* are domains that preferentially bind to binding sites on molecules, such as epitopes, with a higher binding affinity than background interactions between molecules. In the context of the invention, background interactions are interactions with an affinity lower than a K_{D} of 10E-4 M. Preferably, specific binding domains bind with an affinity higher than a K_{D} of about 10E-7 M. Specific binding domains in the proteinaceous molecules of the invention have at least a molecular size allowing their folding into a binding site. At the upper size limit, the binding domains have a molecular size still allowing proper and stable folding, and expression. Typically, domains meeting these size requirements are approximately 25 up to 500 amino acid residues in length, and preferred domains are 40 - 200 amino acid residues in length, and more preferably domains are about the size of a variable domain of a heavy chain of an immunoglobulin ('Vh' or Vhh). For the proteinaceous molecules of the invention, of particular use are specific binding domains present in immune molecules, such as those present in T-cell receptors and immunoglobulins. Especially, a Vh sequence is a preferred specific binding domain in the proteinaceous molecules of the invention. Vh domains are specially suitable for use as a specific binding domain. Vh domains are relatively stable and easy to obtain via various expression systems. Moreover, engineering methods to further improve for example domain stability or solubility are readily available. An available good source for such binding domains consisting of Vh sequences are phage display libraries. Also a good source for such binding domains are natural libraries, synthetic libraries and semi-synthetic libraries.

As said, the specific binding domains in the proteinaceous molecules of the invention are typically separated by at least one linker. Preferably, these linkers are connected with binding domains through peptide bonds. In many instances a simple Gly-Ser linker of 4-15 amino-acid residues may suffice, but if greater flexibility of the amino-acid chain is desired and/or when greater spacing between consecutive domains is desired longer or more complex linkers may be used. Preferred linkers are (Gly₄Ser)ₙ, (GlySerThrSerGlySer)ₙ or any other linker that provides flexibility for protein folding and flexibility for the polypeptide to exhibit its dual or multiple activity, i.e. binding to two or more different binding sites. Additional examples of suitable linkers are the linker sequences connecting domains in human multi-domain plasma proteins. Using linker sequences adapted from multi-domain plasma proteins including immunoglobulins has several advantages. Use of these human amino-acid sequences that are exposed in plasma, in the molecules of the invention may lower the risk for adverse immune responses when applied to human individuals. Moreover, these linker sequences are optimized by natural selection to provide multi-domain proteins required inter-domain flexibility and/or spacing for exerting two or more protein - target interactions simultaneously, involving two or more domains in the multi-domain protein. Examples of such multi-domain plasma proteins comprising inter-domain linkers are vitronectin, fibrinogen, factor V, factor VIII, factor IX, factor X, fibronectin, von Willebrand factor, factor XII, plasminogen, factor H, factor I, C1, C3, beta2-glycoprotein 1, immunoglobulin M, immunoglobulin G. Examples of linkers particularly suitable for covalently connecting domains in the single-chain molecules of the invention are linkers based on amino-acid sequences of hinge regions in immunoglobulins of preferably human origin.

According to the invention the at least two, preferably at least four, most preferably at least six specific binding domains of the proteinaceous molecules of the invention are different or the same binding domains, endowed with binding affinity for at least two different or the same binding sites. It is appreciated that within the context of the current invention binding sites are (parts of) molecules associated with the surface of pathogens or associated with the surface of infected cells of the human body that are infected or altered upon exposure of the body to a pathogen. It is part of the invention that the different binding sites are part of different molecules, or are located on the same molecule, or any combination thereof. Thus, according to the invention the at least two binding sites targeted by the at least two specific binding domains of the proteinaceous molecules of the invention are associated with the surface of pathogens or with the surface of infected cells. In a preferred embodiment of the invention, the different binding sites are co-located at the surface of the same pathogen or co-located at the surface of the same infected cell. Preferred binding sites are binding sites located at pathogen surface molecules or at infected cell surface molecules. Examples of such surface molecules are membrane-anchored glycoproteins, cell surface receptors, cell surface markers, (viral) capsid proteins, on the surface of pathogens, and major histocompatibility complex (MHC) molecules complexed with peptides derived from or from proteins induced by pathogens, on the surface of infected cells.

The term *pathogen* in the context of this application is referring to viruses, bacteria, protozoa, multi-cellular parasites, helminthes, eukaryotic fungi, and other inconvenient micro-organisms, all posing a threat to the health or well-being of an individual colonized by such a pathogen.

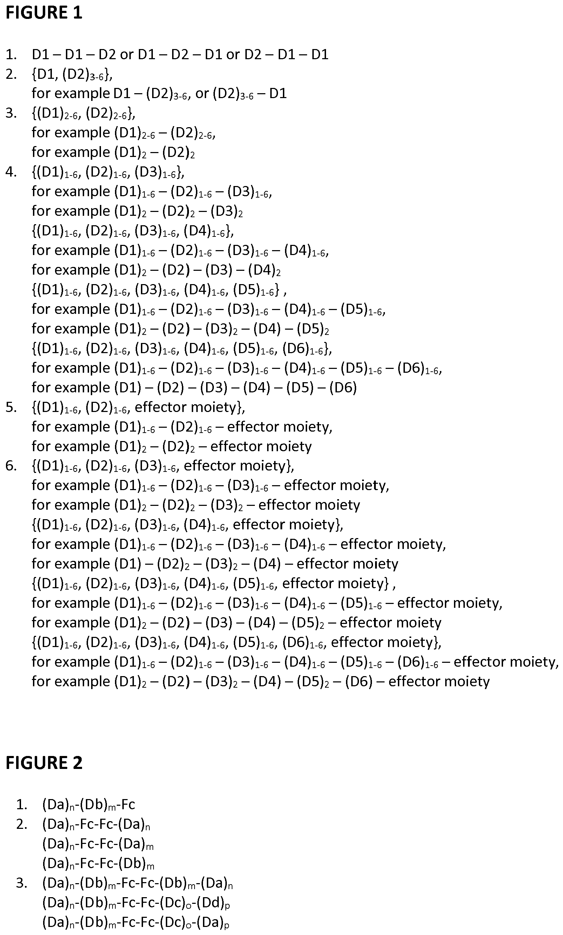

Thus, according to the invention, proteinaceous molecules comprising at least two ,preferably at least four, most preferably at least six specific binding domains are provided ('multi-valent' proteinaceous molecules of the invention) that are particularly suitable for binding to at least two binding sites associated with the surface of pathogens or with the surface of cells infected by a pathogen. In one embodiment, the affinity of the binding molecules for different target binding sites separately, preferably is designed such that Kon and Koff are optimally selected for efficient and sufficient binding of the binding molecules through one of the at least two different binding domains. Thus, the specificity of the proteinaceous molecules of the invention is even further increased by increasing their avidity for binding to multiple binding sites on pathogens or on infected cells. The avidity is preferably further increased by incorporating multiple copies, preferably two to six copies, of at least one of the at least two different binding domains in the proteinaceous molecules ('multi-valent' proteinaceous molecules of the invention). Figures 1-3 give a number of preferred molecular designs of proteinaceous molecules of the invention. It is appreciated that at least one copy of each of the at least two different specific binding domains of the proteinaceous molecules of the invention must bind to their respective binding sites. Of course, it is preferred that two or more of the copies bind simultaneously, and most preferably, all copies of a binding domain present in the proteinaceous molecule bind simultaneously. In the above described methods the likelihood of targeting only infected cells increases as the number of different binding sites for a pathogen increases. Inversely the likelihood of finding a target expressing all different targets decreases. It is therefore preferred to carefully design the molecules such that a balance between these counteracting mechanisms is achieved.

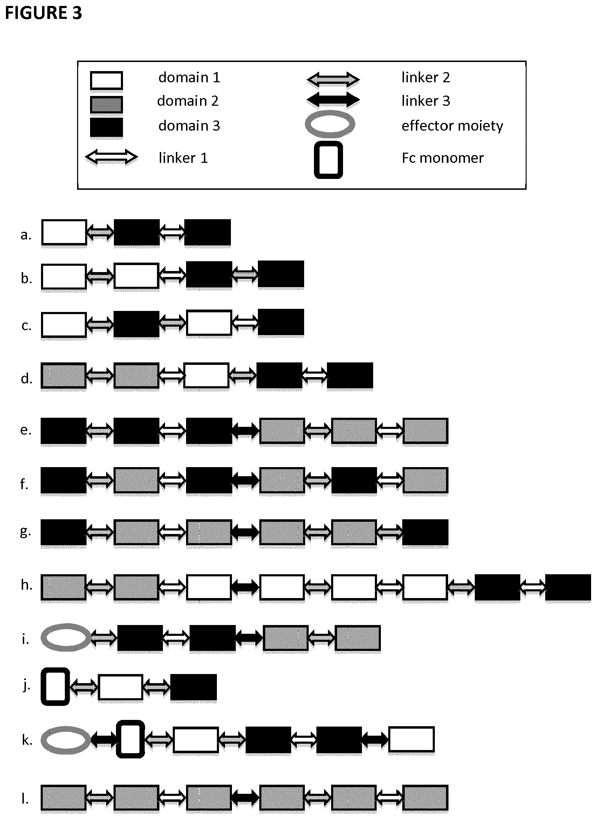

In an embodiment of the invention a proteinaceous molecule is provided, comprising at least three specific binding domains preferably for different binding sites separated from each other by at least one linker. In such embodiments one may also employ binding domains targeting binding sites on different pathogens, thereby creating one molecule capable of treating several infections at once.
It is preferred that proteinaceous molecules of the invention comprise the minimal number of different specific binding domains providing the specificity for pathogens or for infected cells exposing pathogen-associated binding sites (preferably in the context of MHC). It is then also preferred that the proteinaceous molecules of the invention comprise the minimal number of copies of each of the different specific binding domains, required for providing the desired affinity. These preferred proteinaceous molecules of the invention regarding specificity and affinity, are selected from libraries of possible proteinaceous molecules with varying numbers of different binding domains, varying numbers of copies of each of the different domains, and different domain topologies possible with the varying numbers of different domains and copies. Preferably, proteinaceous molecules of the invention comprise two or three different binding domains, but also mono-specific proteinaceous molecules are provided by the invention. Preferably, proteinaceous molecules of the invention comprise four to twelve copies of one binding domain or one to six copies of each of the different binding domains. Thus a typical proteinaceous molecule of the invention comprises two different binding domains A, B with three copies of each domain, with domain topology A-B-A-B-A-B. See for examples of preferred proteinaceous molecules regarding number of different domains, copies of domains and topologies, Figure 1, Figure 2 and Figure 3. Repetitive proteinaceous structures are sometimes difficult to express. By selecting (modestly) different binding domains specific for the same molecule, or even for the same binding site on the molecule, expression issues with repetitive structures are largely diminished. These expression problems are further addressed by selecting different linkers for connecting consecutive domains. Thus, an example of a typically preferred molecule of the invention has the following structure: A-linker1-B-linker2-A'-linker3-B'-linker1-A"-linker2-B". See Figure 3 for other examples of proteinaceous molecules of the invention.

Thus, in a preferred embodiment according to the invention, proteinaceous molecules comprising at least three different specific binding domains are provided that are particularly suitable for binding to at least three different binding sites associated with the surface of pathogens or associated with the surface of cells infected by pathogens.

Also provided according to the invention is a proteinaceous molecule comprising at least two specific binding domains for the same binding site separated by at least one linker wherein said proteinaceous molecule comprises a single polypeptide chain, for use in the treatment of an infectious disease. See Figure 3 for an example of such a mono-specific proteinaceous molecule of the invention. Other examples of a typically preferred molecule of the invention have the following structure: A-linker1-A'-linker2-A" or A-linker1-A-linker2-A-linker1-A-linker3-A. A, A' and A" represent binding domains having (slightly) different sequences but recognizing the same epitope. Preferably, such a mono-specific multivalent proteinaceous molecule of the invention comprises two to ten specific binding domains for the same binding site. In an even more preferred embodiment of the invention, such a mono-specific proteinaceous molecule of the invention comprises four to six specific binding domains for the same binding site. Preferably, the specific binding domain of such a mono-specific proteinaceous molecule of the invention binds with an affinity higher than a K_{D} of about 10E-7 M. According to the invention, the affinity of a single specific binding domain of the mono-specific multi-valent proteinaceous molecules is high enough for binding of the mono-specific multi-valent proteinaceous molecules of the invention to a target binding site on a pathogen or on an infected cell (e.g. a pathogen derived peptide epitope presented in the context of MHC at the surface of the infected cell) already through interaction of a single specific binding domain. A comparable approach for inducing apoptosis in tumor cells has been disclosed in WO12/091564 from the same applicant incorporated herein by reference.

In a preferred embodiment, the proteinaceous molecules of the invention comprise specific binding domains comprising at least one Vh domain. More preferably, all two, three or more specific binding domains in the proteinaceous molecules of the invention are Vh domains. Thus, a proteinaceous molecule according to the invention is a proteinaceous molecule wherein at least one specific binding domain is a Vh domain. Preferable Vh domains are human Vh domains.

In a preferred embodiment of the invention binding sites targeted by the proteinaceous molecules of the invention are located at the surface of the same pathogen or the same infected cell. It is preferred that binding of proteinaceous molecules of the invention to target pathogens or to target infected cells induces target pathogen or target infected cell phagocytosis or lysis pathways. Also incorporated in the invention are proteinaceous molecules that are internalized by the infected cell. In a preferred embodiment the infected cells go into apoptosis as a result of said internalization or by cross-linking several proteins on the surface of the infected cell.

In one preferred embodiment, the proteinaceous molecules of the invention further comprise at least one effector moiety, linked to the polypeptide chain comprising the specific binding domains. Effector moieties preferably improve the potency of a therapeutic molecule and/or increase the efficacy of a therapeutic molecule. It is part of the current invention that effector moieties are covalently bound to proteinaceous molecules of the invention via peptide bonds, and preferably via a linker. Alternatively, as part of the invention effector moieties are linked to the proteinaceous molecules applying any other suitable linker chemistry known in the art. Yet in another embodiment, the proteinaceous molecules of the invention comprise specific binding domains for binding sites on effector moieties. An advantage of such binding molecules of the invention is the provided flexibility in the order of binding events. Proteinaceous molecules of the invention can first bind to target binding sites on pathogens or on infected cells, followed by binding to an effector moiety exposed to the proteinaceous molecules localized on the pathogens or on the infected cells. Such a proteinaceous molecule of the invention is for example used for the treatment of cervical cancer related to human papilloma virus infection of the tumor cells.

Preferred effector moieties according to the invention are numerous, e.g. toxins, statins, apoptin, chelated radioactive metal ions, radioactive iodine. Other suitable effector moieties according to the invention are ricin A, gelonin, saporin, interleukin-2, interleukin-12, viral proteins E4orf4 and NS1, and non-viral cellular proteins HAMLET, TRAIL and mda-7 of which the latter five can, like apoptin, specifically induce apoptosis in aberrant cells after internalization of the proteinaceous molecules of the invention comprising at least one of such effector moieties.

When proteinaceous molecules of the invention are designed to first bind to a target pathogen or to a target infected cell, followed by internalization, the effector moiety can then subsequently have its intracellular (cytotoxic) function. It is preferred that such an effector moiety has a contribution to the specificity of the cytotoxic effect. Therefore it is preferred to use as an effector moiety a molecule that induces cell death in pathogens or in infected cells, but not in normal cells Thus the invention provides a proteinaceous molecule, further comprising an effector moiety.

Particularly suitable and preferred specific binding domains according to the invention are domains based on Vh sequences. Thus the invention also provides proteinaceous molecule comprising at least two Vh domains. Examples of such molecules of the invention are provided in Figure 1, Figure 2 and Figure 3. In a preferable embodiment these Vh domains are derived from human Vh sequences. It is appreciated that Vh domains as such are already relatively stable. Still, stability and solubility of human Vh domains can be further improved by engineering approaches known in the art. Particularly suitable for the purpose is applying a process referred to as 'camelization' of the human Vh sequence. Now, selected amino acid residues in the human Vh sequence, not contributing to the binding specificity and affinity of the domain, are replaced for amino acid residues present at the corresponding sites of llama Vh domains. Preferred amino acid substitutions contributing to improved stability/solubility are Glu6Ala, Ala33Cys, Val37Phe, Gly44Glu, Leu45Arg, Trp47Gly, Ser74Ala, Arg83Lys, Ala84Pro, Trp103Arg or Leu108Gln. Thus the invention also provides proteinaceous molecule comprising camelized human Vh domains with improved stability and/or solubility.

Other functions that may be introduced in the proteinaceous molecules of the invention may have to do with improved half-life (e.g. human serum albumin (HSA) can be included, or one or more binding domains binding to a binding site in HSA can be included) or with complement activation (Fc monomer of immunoglobulins can be included; in this case the molecules according to the invention may dimerize). Other functionalities that can be incorporated are cytokines, hormones, Toll-like receptor ligands, (activated) complement proteins, etc.

Thus the invention also provides a proteinaceous molecule comprising at least two Vh domains and an Fc monomer. The invention also provides a dimeric proteinaceous molecule, comprising two proteinaceous molecules dimerized through two Fc monomers. Proteinaceous molecules comprising immunoglobulin CH3 domains are also part of the invention. Similar to Fc monomers, the CH3 domain can serve as a dimerization domain. Homo-dimeric as well as hetero-dimeric proteinaceous molecules are part of the invention. Homo-dimeric binding molecules for example comprise dimerized Fc monomers with identical arms. The heterogeneity of hetero-dimeric proteinaceous molecules of the invention originates from the two Fc monomers in the hetero-dimer, differing in the type, number and/or topology of their respective specific binding domains, linkers and/or effector moieties. Thus, in one embodiment the invention also provides a hetero-dimeric molecule comprising two different proteinaceous molecules. The two different proteinaceous molecules are then dimerized through their respective Fc monomers. Upon applying preferred pairing biochemistry, hetero-dimers are preferentially formed over homo-dimers. For example, two different Fc monomers are subject to forced pairing upon applying the "knobs-into-holes" CH3 domain engineering technology as described [Ridgway et al., Protein Engineering, 1996]. An advantage of the proteinaceous molecules of the invention comprising dimerized Fc monomers is the localization of phagocytosis activity and/or cell lytic activity at the surface of pathogens or infected cells to which these proteinaceous molecules bind. These activities can enhance the deleterious effects on pathogens or on infected cells, induced by the proteinaceous molecules of the invention specifically bound to these pathogens or infected cells. A further advantage of such hetero-dimeric proteinaceous molecules of the invention is their increased spatial flexibility regarding the different/differently located specific binding domains in the two arms.

In one embodiment of the invention, binding molecules are provided comprising one or multiple copies of each of binding domains specific for binding sites on pathogens or on infected cells. Infection-induced cellular aberrancies such as some cancers are manifested by the presence of unique pathogen-associated molecular patterns on the aberrant cell surface. It is thus one of the preferred embodiments of the invention that the at least two different binding sites targeted by proteinaceous molecules of the invention are all uniquely located on infected aberrant cells. It is thus also preferred that these at least two different binding sites are not at all present on normal cells, or that at least one of the targeted binding sites is uniquely present at the surface of the targeted pathogen or targeted infected cell and not present on normal cells.

Thus, in one embodiment the invention provides an immunoglobulin molecule that is specifically binding to two different binding sites (so-called bi-specific immunoglobulins of the invention) associated with the cell surface of aberrant cells altered upon infection. Preferred immunoglobulins of the invention are antibodies, but fragments and/or derivatives such as Fab and/or ScFv can also be used. Even more preferred immunoglobulins of the invention are antibodies of the immunoglobulin G (IgG) type.

In a preferred embodiment the invention provides a bi-specific immunoglobulin molecule provided with a toxic moiety.

Thus, in a preferred embodiment, a proteinaceous molecule according to the invention is provided for use in the treatment of an infectious disease. And thus, in an additionally preferred embodiment, a proteinaceous molecule according to the invention is provided for use in the treatment of a disease related to infected (aberrant) cells.

For administration to subjects the proteinaceous molecules according to the invention must be formulated. Typically these proteinaceous molecules will be given parentally. For formulation simply saline for injection may suffice. For stability reasons more complex formulations may be necessary. The invention contemplates lyophilized compositions as well as liquid compositions, provided with the usual additives. Thus the invention provides a pharmaceutical formulation comprising a proteinaceous molecule according to any of the embodiments of the invention and suitable excipients.

The dosage of the proteinaceous molecules according to the invention must be established through animal studies and clinical studies in so-called rising-dose experiments. Typically, the doses will be comparable with present day antibody dosages (at the molar level, the weight of the invented molecules may differ from that of antibodies). Typically, such dosages are 3-15 mg/kg body weight, or 25-1000 mg per dose.

It is anticipated that in the field of for example virology the proteinaceous molecules of the invention will replace current single agents binding to a single binding site. In addition, especially in the more difficult to treat infections the first applications of the proteinaceous molecules according to the invention will (at least initially) probably take place in combination with other treatments (standard care). Of course, the invention also provides proteinaceous molecules for use in novel or first treatments of any infection, for which current treatments are not efficient enough or for which currently no treatment options are available. Thus, for example the invention also provides a pharmaceutical composition comprising an invented proteinaceous molecule and a conventional cytostatic and/or tumoricidal agent. Moreover, the current invention also provides a pharmaceutical composition comprising an invented proteinaceous molecule for use in an adjuvant treatment of an infection. Additionally, the current invention also provides a pharmaceutical composition comprising an invented proteinaceous molecule for use in a combination chemotherapy treatment of cancer related to an infection. Examples of chemotherapeutical treatments that are combined with the pharmaceutical composition of the current invention are etoposide, paclitaxel, doxorubicin and methotrexate.

The invention of course also comprises a nucleic acid molecule encoding a proteinaceous molecule according to any of the embodiments of the invention. The molecules according to the invention can be produced in prokaryotes as well as eukaryotes. The codon usage of prokaryotes may be different from that in eukaryotes. The nucleic acids according to the invention can be adapted in these respects. Also, elements that are necessary for secretion may be added, as well as promoters, terminators, enhancers etc. Also, elements that are necessary and/or beneficial for the isolation and/or purification of the proteinaceous molecules may be added. Typically, the nucleic acids according to the invention are provided in an expression vector suitable for the host in which they are to be produced. Choice of a production platform will depend on the size of the molecule, the expected issues around protein folding, whether additional sequences are present that require glycosylation, expected issues around isolation and/or purification, etc. For example, whether or not specific binding domains of the invention comprise disulphide bonds will guide the selection of the preferred production platform. Thus, typically nucleic acids according to the invention are adapted to the production and purification platform in which the proteinaceous molecules according to the invention are to be produced. Thus, the invention provides a vector comprising a nucleic acid molecule encoding a proteinaceous molecule according to the invention. For stable expression in an eukaryote it is preferred that the nucleic acid encoding the proteinaceous molecule according to the invention is integrated in the host cell genome (at a suitable site that is not silenced). In one embodiment the invention therefore comprises: a vector comprising means for integrating the nucleic acid in the genome of a host cell. The invention further comprises the host cell or the organism in which the proteinaceous molecule encoding nucleic acid molecule is present and which is thus capable of producing the proteinaceous molecule according to the invention. Thus, in a preferred embodiment the invention comprises a cell comprising a nucleic acid molecule according to the invention, preferably integrated in its genome and/or a vector according to the invention, comprising a nucleic acid molecule encoding a proteinaceous molecule according to the invention.

Included in the present invention is also a method for producing a proteinaceous molecule according to the invention, comprising culturing a cell according to the invention, comprising a nucleic acid molecule encoding a proteinaceous molecule according to the invention, preferably integrated in the cell's genome and/or a vector according to the invention, comprising a nucleic acid molecule encoding a proteinaceous molecule according to the invention, allowing for expression of the proteinaceous molecule and separating the proteinaceous molecule from the culture.

The pharmaceutical compositions according to the invention will typically find their use in the treatment of infections and of forms of cancer where the pathogen-associated antigen binding sites of the preferred proteinaceous molecules of the invention are presented by the tumors. Examples of such binding sites are complexes of MHC and peptides derived from HPV E6 protein, or from Epstein-Barr virus proteins exposed by Hodgkin's lymphoma cells. It is easy using binding domains according to the invention to identify pathogens or to identify tumors that present pathogen associated antigen(s). This can be done *in vitro* or *in vivo* (imaging).

Typical proteinaceous molecules of the invention according to any of the aforementioned embodiments are provided and exemplified by the binding molecules outlined in this section, in Figure 1, Figure 2 and Figure 3, and by the examples provided below and in the Examples section. Thus the invention provides a proteinaceous molecule according to Figure 1, Figure 2 or Figure 3.

### Detailed description.

An *Fc fragment* is a dimer composed of two linked Fc monomers. The two Fc monomers are covalently bound in the Fc fragment, preferably via one or more disulphide bonds between cystein residues, or are non-covalently bound. An Fc monomer is commonly composed of two or three constant domains C, commonly referred to as CH2-CH3 or CH2-CH3-CH4, respectively. More specifically, any functional fragment of an Fc fragment is part of the invention. An example of such a functional fragment of an Fc fragment is the CH2 domain or the CH3 domain.

A further aspect of the invention relates to a method for providing the binding molecules according to the invention. As described herein above, it typically involves providing a nucleic acid construct encoding the desired binding molecule. Said nucleic acid construct can be introduced, preferably via a plasmid or expression vector, into a prokaryotic host cell and/or in a plant cell and/or in a eukaryotic host cell capable of expressing the construct. In one embodiment, a method of the invention to provide a binding molecule comprises the steps of providing a host cell with one or more nucleic acid(s) encoding said binding molecule capable of recognizing and binding to multiple binding sites, and allowing the expression of said nucleic acids by said host cell.

Binding molecules of the invention are for example expressed in plant cells, eukaryotic cells or in prokaryotic cells. Non-limited examples of suitable expression systems are tobacco plants, *Pichia pastoris, Saccharomyces cerevisiae.* Also cell-free recombinant protein production platforms are suitable. Preferred host cells are bacteria, like for example bacterial strain BL21 or strain SE1, or mammalian host cells, more preferably human host cells. Suitable mammalian host cells include human embryonic kidney (HEK-293) cells or Chinese hamster ovary (CHO) cells, which can be commercially obtained. Insect cells, such as S2 or S9 cells, may also be used using baculovirus or insect cell expression vectors, although they are less suitable when the polypeptides according to the invention include elements that involve glycosylation. The produced binding molecules according to the invention can be extracted or isolated from the host cell or, if they are secreted, from the culture medium of the host cell. Thus, in one embodiment a method of the invention comprises providing a host cell with one or more nucleic acid(s) encoding said binding molecule, allowing the expression of said nucleic acid(s) by said host cell. In another preferred embodiment a method of the invention comprises providing a host cell with one or more nucleic acid(s) encoding two or more different binding molecules allowing the expression of said nucleic acids by said host cell. For example, in one embodiment, nucleic acids encoding for two or more different binding molecules all comprising an Fc monomer are provided, enabling isolation of multiple single-chain binding molecules, and/or enabling isolation of homo-dimers and/or hetero-dimers formed through Fc dimerization. Methods for the recombinant expression of (mammalian) proteins in a (mammalian) host cell are well known in the art.

As will be clear, a binding molecule of the invention finds its use in many therapeutic applications and non-therapeutic applications, e.g. diagnostics,. Proteinaceous molecules of the invention suitable for diagnostic purposes are of particular use for monitoring the expression levels of molecules exposing binding sites on pathogens or on cells infected by pathogens, that are targeted by proteinaceous molecules of the invention applied for their therapeutic use. In this way, it is monitored whether the therapy remains efficacious or whether other proteinaceous molecules of the invention targeting one or more different binding sites on the pathogen or on the infected aberrant cells should be applied instead, in case the expression levels of the first targeted binding sites are below a certain threshold. Binding molecules of the invention may also be used for the detection of (circulating) tumor cells related to infection. Or for the target-pathogen- or target-cell specific delivery of cytotoxic compounds, or for the delivery of immune-stimulatory molecules.

Accordingly, also provided is the use of a binding molecule according to the invention as medicament. In another aspect, the invention provides the use of a binding molecule for the manufacture of a medicament for the treatment of infections, aberrancies such as cancer related to infections,. Viral infections that can be treated with the invented molecules and compositions include, but are not limited to, Hepatitis viruses (in particular HCV), RSV, HIV, influenza, herpesviruses and humanpapilloma viruses.

In one embodiment, proteinaceous molecules of the invention comprise binding domains mimicking pattern recognition receptors (PRRs) present on the cells of the body. These PRRs are part of the body's defense mechanism against invading pathogens. The PRRs recognize and bind to broadly shared molecular patterns specifically associated with (classes of) pathogens and not with molecules of the host. Examples of PRRs are the extra-cellular and intra-cellular Toll-like receptors (TLR) 1-13. Proteinaceous molecules of the invention comprising binding domains mimicking binding capacities of one or more different PRRs are particularly suitable for binding to pathogens exposing the at least one or more different binding sites for this/these PRR(s). An example is a proteinaceous molecule of the invention comprising at least one copy of a binding domain mimicking TLR-2, for binding to gram-positive bacteria exposing a lipoprotein binding site for TLR-2. Of course, in alternative binding molecules of the invention binding domains mimicking binding capacities of PPRs are also combined with different binding domains binding to other pathogen-associated binding sites.

Antibody fragments of human origin can be isolated from large antibody repertoires displayed by phages. One aspect of the invention, known by the art, is the use of human antibody phage display libraries for the selection of two or more human antibody fragments specific for two or more selected different binding sites, e.g. epitopes. These antibody fragments usually display low affinity. It is an important aspect of the current invention that binding domains specific for pathogens or pathogen-related antigen on aberrant cells are selected for their relatively high affinity. A method is provided that allows the generation of high avidity antibody domain chains able to bind and exert the modulating biological activity in a specific and efficient manner. An aspect of the present invention is the development of a binding molecule comprising multiple binding domains. That is to say, preferably a human Vh domain, capable of binding to a certain binding site combined with a second, third, fourth, and so on copy of an identical binding domain (*multi-valency*), and at least one copy of one or more different human Vh domains with each different human Vh domain capable of binding to a separate binding site (*multi-specificity*). In this way, avidity regarding the first binding site and, if multiple binding domains are applied specific for a second, third, fourth, and so on binding site, avidity regarding this second, third, fourth, and so on binding site is enhanced.

Thus, a proteinaceous molecule is provided comprising at least two copies of a binding domain specific for a binding site functionally connected with at least one copy of a different binding domain specific for a different binding site or with a different affinity for the same binding site. Preferably, these different binding domains are functionally connected to each other via peptide bonds between amino-acid residues flanking the binding domains, providing a linear single chain proteinaceous molecule (Figure 1). It is also part of the invention that the binding domains are linked together via bonds and/or binding interactions other than covalent peptide bonds between amino acid residues in a linear sequence. Alternative methods for linking proteinaceous molecules to each other are numerous and well known to those skilled in the art of protein linkage chemistry. Protein linkage chemistry not based on peptide bonds in a single chain amino acid sequence can be based on covalent interactions and/or on non-covalent interactions.
A multi-specific proteinaceous molecule in a monovalent or multivalent binding molecule form of the invention capable of modulating a biological process such as an infection is for example composed of at least copies of two different human Vh domains or functional fragments thereof which are multimerized at the DNA level in order to obtain a single-chain polypeptide construct upon expression.

Isolated human Vh domains usually do not meet the standards for stability and efficient expression that are required by the field. They tend to be unstable, poorly soluble and poorly expressed. A process called "*camelisation*" may be used to convert human Vh into more stable antibody fragments.

The human antibody germ-line region Vh-3 displays high homology with antibody Vh fragments of llamas. Llamas have two types of antibodies, those composed of heavy and light chains, and antibodies that only contain heavy chains. These heavy-chain only antibodies bind antigens similar to classical antibodies composed of heavy and light chains. The smallest functional llama antibody binding domain, the Vhh domain, also called (single) domain antibodies ((s)dAb), have been shown to be expressed well and may bind antigen with high affinity. In addition, it has been shown that some of the characteristics, such as ease of expression and stability, of llama sdAb can be transferred to e.g. human Vh by replacing a few amino acids in the human Vh for those of llama Vhh. Antibody molecules with multi-specificity can then be generated by ligation of one or more copies of several different "camelised" human Vh domains each with affinity for different binding sites, into one single molecule. Moreover, high avidity antibody molecules can then be generated by ligation of several of the camelised human Vh domains binding to the same binding site, into one single molecule.
For each of the at least two binding sites the molecules of the invention comprise one to twelve and more preferably one to six and even more preferably one to three camelised human Vh domains interspersed by short linkers, for example short Gly-Ser linkers, and connected through peptide bonds to the camelised human Vh domains interspersed by short linkers, specific for the other target binding sites of the binding molecules. In another embodiment, for at least one of the at least two binding sites the molecules of the invention comprise preferably four to six camelised human Vh domains interspersed by short linkers, herewith providing the molecules with the capacity to cross-link four to six target molecules exposing this targeted binding site. In an even more preferred embodiment, this cross-linking of molecules induces apoptosis in infected cells expressing surface molecules (e.g. MHC - pathogen-derived antigen peptide complex) exposing the targeted binding site for the four to six binding domains.

Compared to binding molecules specific for a single binding site, the proteinaceous molecules of the invention have amongst others the following advantages regarding efficacy and specificity. The proteinaceous binding molecules of the invention have an increased specificity for infected aberrant cells by targeting multiple binding sites specific for the aberrant cell simultaneously and/or by targeting combinations of binding sites unique to the aberrant cell simultaneously. In this way, aberrant cells are targeted more efficiently, avoiding (excessive) targeting of healthy cells, and thus lowering the risk for toxic and undesired side-effects significantly. This high specificity for infected aberrant cells is achieved with proteinaceous molecules of the invention bearing relatively low affinity for binding sites present on both aberrant cells and healthy cells, while bearing relatively high avidity for aberrant cells exposing a combination of different binding sites unique to the aberrant cells. Below, examples are provided for these combinations of binding sites that provide suitable therapeutic targets for the molecules of the invention. Moreover, with the multi-specific proteinaceous molecules of the invention, difficult to target and/or difficult to reach aberrant cells have a higher chance of being 'hit' by at least one of the binding domains, thereby providing at least in part the therapeutic activity and increasing the success rate when compared to single molecule / single target therapies.

Examples of various preferred domain topologies in the proteinaceous molecules of the invention, as exemplified below, are provided in Figure 1, Figure 2 and Figure 3. For example, a proteinaceous molecule of the invention that is suitable for specifically targeting aberrant B-cells in Epstein-Barr virus (EBV) infection has the following characteristics: four to six binding domains endowed with high affinity for the infected B-cell specific EBV antigen LMP-1 and/or LMP-2A and/or LMP-2B, linked to one or two binding domains endowed with low affinity for one or more of the adhesion molecules LFA1, CD54, and/or CD58 and/or B-cell activation markers CD23, CD39, CD40, CD44, and/or HLA class II, specific for the infected B-cells. The affinity for LFA1, CD54, CD58, CD23, CD39, CD40, CD44, and/or HLA class II is then selected to be so low that no binding occurs to healthy low-expressing non-infected B-cells lacking an EBV-specific antigen LMP. These exemplified proteinaceous molecules of the invention are highly specific for the infected aberrant cells, compared to the healthy (neighboring/circulating) cells. Low affinity and avidity for the proteins also present on the non-infected B-cells prevents binding of the binding molecules to healthy cells. High avidity for the infected B-cells exposing the LMP receptors directs the binding molecules to the aberrant cells. Subsequently, avidity, and thus specificity of the binding molecules is even increased due to sequential binding of the low-affinity / low-avidity binding domains specific for the proteins specific for the B-cells. Therefore, in a preferred embodiment, the desired high specificity for infected aberrant cells and concomitant high efficacy regarding infected cell eradication, leaving healthy cells in essence unaltered, of the proteinaceous molecules of the invention, are tunable ('mix & match' approach) by selecting for, for example:
i) optimal target binding sites,
ii) optimal number of different binding sites,
iii) optimal number of binding domains for each selected binding site,
iv) optimal domain topologies,
v) optimal affinity of each binding domain,
vi) optimal avidity for each binding site and for the proteinaceous molecule as a whole,
vii) optimally facilitating cellular uptake of the proteinaceous molecules of the invention (for example, when the binding molecule comprises apoptin),
viii) optimally facilitating clustering of molecular complexes of targeted surface molecules with bound proteinaceous molecules of the invention at the outer membrane surface of infected cells (for example, when the targeted binding sites on surface molecules are complexes of MHC 2 with pathogen derived peptides).

### Abbreviations used

Ab, antibody; CH, constant domain of the heavy chain of an antibody; CHO, Chinese hamster ovary; DAMPs, damage associated molecular patterns; HEK, human embryonic kidney; HPV, human papilloma virus; IEP, iso-electric point; Ig, immunoglobulin; MAGE, melanoma-associated antigen; MHC, major histocompatibility complex; PAMPs, pathogen associated molecular patterns; RA, rheumatoid arthritis; sc-Fv, single-chain variable fragment; V_{HH} or sdAb, single domain antibodies; VH, Vh or V_{H}, variable amino-acid sequence of an antibody heavy domain.

### Examples

Examples of at least two different binding sites each targeted in a monovalent or multivalent manner by proteinaceous molecules of the invention comprising at least two different binding domains, such as depicted in Figure 1, Figure 2 and Figure 3, are provided in the specification and in the examples, below.

### EXAMPLE 1

Non-exhaustive examples of proteinaceous molecules of the invention comprising binding domains binding to at least two different binding sites which are each targeted in a monovalent or multivalent manner by the different binding domains, with binding domain topologies as outlined for example in Figure 1, Figure 2 and Figure 3, are:
Proteinaceous molecules of the invention comprising binding domains binding to
a. one or more epitopes in human immunodeficiency virus-1 (HIV-1) envelope protein gp41, and to one or more epitopes in HIV-1 envelope protein gp120, for the treatment of HIV-1 infection or acquired immune-deficiency syndrome, or for opportunistic infection prophylaxis, for example by neutralizing HIV-1;
b. one or more epitopes in human immunodeficiency virus-1 (HIV-1) envelope protein gp120, for example an epitope in the CD4 binding site of gp120 and an epitope in the V3 region of gp120, for the treatment of HIV-1 infection or acquired immune-deficiency syndrome, or for opportunistic infection prophylaxis, for example by neutralizing HIV-1;
c. one or more epitopes in human immunodeficiency virus-1 (HIV-1) envelope protein gp41, for example an epitope encompassing any of the gp41 amino-acid sequences 656-671, 659-673, 660-667, 660-670, 661-670, 662-ELDKWA-667 or 662-ELDKWAS-668, for the treatment of HIV-1 infection or acquired immune-deficiency syndrome, or for opportunistic infection prophylaxis, for example by neutralizing HIV-1;
d. at least one epitope encompassing any of the gp41 amino-acid sequences 656-671, 659-673, 660-667, 660-670, 661-670, 662-ELDKWA-667 or 662-ELDKWAS-668 of HIV-1 and/or to at least one epitope in the conserved V3 region of gp120 of HIV-1 and/or to at least one epitope in the conserved CD4 binding site of gp120 of HIV-1, for the treatment of HIV-1 infection or acquired immune-deficiency syndrome, or for opportunistic infection prophylaxis, for example by neutralizing HIV-1;
e. one or more epitopes in two or more antigens, or to two or more binding sites in a single antigen, exposed by for example lipid-A, lipo-polysaccharides, toxins, Rabies, Hepatitis virus, Herpes virus, Rubella virus, Varicella-zoster virus, Staphylococcus, Streptococcus, Hemophilus, Actinomycetes, Pseudomonas, Neisseria, for the treatment of diseases or health problems related to infections by these pathogens and/or related to infections accompanied by the exposure to these molecules;
f. one or more epitopes in two or more antigens, or to two or more binding sites in a single antigen for which the antigen is (part of) an agent of use in biological warfare, including toxins, plague, smallpox, anthrax, hemorrhagic fever virus, ricin, for the prevention of devastating health effects upon exposure to these agents;
g. one or more conserved epitopes in the F subdomain of influenza A virus hemagglutinin glycoprotein, for the neutralization of influenza A virus comprising any of the (sixteen) known hemagglutinin subtypes of group 1 and group 2 influenza A viruses, for use as a universal prophylactic or therapeutic flu vaccine;
h. one or more conserved epitopes in the F subdomain of influenza A virus hemagglutinin glycoprotein and/or to one or more conserved epitopes in the virus' M protein and/or to one or more conserved epitopes in the virus' neuramidase protein, for the treatment of influenza A virus infection, for use as a universal flu vaccine;
i. one or more epitopes in the CD4-binding site in the gp120 subunit of human immunodeficiency virus type 1 (HIV-1)'s trimeric gp120-gp41 envelope spike and to one or more epitopes in the membrane-proximal external region (MPER) of gp41, for neutralizing HIV-1 strains;
j. two or more epitopes in a capsular polysaccharide of *Streptococcus pneumonia,* or to one or more epitopes in two or more different capsular polysaccharides of *Streptococcus pneumonia,* for the protection against infection (prophylaxis) or for the treatment of infection;
k. one or more epitopes in the Epstein-Barr virus proteins Epstein-Barr nuclear antigen 1 and/or in latent membrane protein 1 and/or in latent membrane protein 2, for the treatment of Hodgkin's lymphoma associated with Epstein-Barr virus infection;
l. two or more epitopes in soluble IL-1-binding proteins produced by cowpox or vaccinia, to prevent binding to secreted IL-1 in the infected body, and thus to prevent the inhibitory activity on the inflammatory response of the body;
m. two or more epitopes in TNF-receptor mimic of the Shope fibroma virus, for inhibiting the binding of the TNF-receptor mimic to TNF in the infected body, thereby inhibiting the anti-inflammatory activity of the TNF-receptor mimic;
n. two or more epitopes in Epstein-Barr virus BCRF1 protein, for inhibiting the stimulatory effect of this human IL-10 analog BCRF1 on production of T-helper 2 cells. Stimulating T-helper 2 cells simultaneously down-regulates T-helper 1 activation, thereby inhibiting T-helper 1 inflammatory response beneficial for infection suppression;
o. at least two binding sites in the complex of peptide E2(614-622) of Hepatitis C virus with HLA-A2, for the treatment of Hepatitis C virus infection;
p. at least two conserved (conformational) epitopes on surface E2 glycoprotein present in all major genotypes (1a, 1b, 2a, 2b, 3a, 4, 5, 6) of Hepatitis C virus, for treatment of Hepatitis C virus infections;
q. one or more epitopes in EBV receptors LMP-1, LMP-2A, LMP2B on infected B-cells and one or multiple copies of binding domains neutralizing EBV derived interleukin-10 homologue BCRF-1 and/or EBV derived BCL-2 homologue BHRF-1 and/or EBV derived C-FMS receptor homologue BARF-1, for the eradication of (primary) EBV-infected cells;
r. one or more epitopes in EBV surface molecules gp220 and/or gp340 and/or gp350, for the eradication of EBV from the body;
s. the HLA B8 restricted epitope from EBV nuclear antigen 3, FLRGRAYGL, complexed with MHC I, and one or more domains binding to a second surface molecule specific for EBV infected cells, for the clearance of EBV infected cells;
t. one or more IgE binding sites on a food allergen, for the prevention of an allergic reaction by neutralizing the IgE binding sites.

Of particular interest are of course combinations of surface molecules exposed by pathogens or by infected aberrant cells exposing pathogen-specific antigens. Targeting binding sites on one of such exposed molecules unique to the infected aberrant cell by proteinaceous molecules of the invention would already provide high specificity for aberrant cells over healthy cells not expressing the pathogen-specific antigen. Specificity and efficacy is then even further improved when binding domains are combined in proteinaceous molecules of the invention, that target binding sites in two or more pathogen-specific antigens uniquely exposed by the infected aberrant cell. Such molecules of the invention provide even an higher specificity than molecules of the invention targeting two different antigens which are co-expressed on aberrant cells, with one of the two antigens also expressed on healthy cells. Combining binding domains with relatively high affinity for pathogen-specific antigens exposed by aberrant cells with binding domains with relatively low affinity for other surface markers of the particular infected cell type further improves the specificity of the proteinaceous molecules of the invention for the aberrant cells. Especially when the affinity for the surface markers specific for the type of cells is below a certain threshold prohibitive for binding of the proteinaceous molecules of the invention to healthy cells via binding interactions with these surface markers alone.

Target binding sites suitable for specific targeting of infected aberrant cells by proteinaceous molecules of the invention are combinations of pathogen-derived antigen peptides complexed with MHC molecules. Examples of T-cell epitopes of the E6 and E7 protein of human papilloma virus, complexed with indicated HLA molecules, are provided below. Any combination of pathogen-derived T-cell epitope and bound HLA molecule provides a specific target on infected cells for molecules of the invention. An example of an infected cell is a keratinocyte in the cervix infected by human papilloma virus (HPV), presenting T-cell epitopes derived from for example E6 or E7 protein, in the context of MHC.

For example, the invention provides binding molecules that comprise low-affinity binding domains binding to immune-reactive thrombomodulin expressed on suprabasal spinous layer keratinocytes, low-affinity binding domains binding the squamous cell-marker SPRR1 and high-affinity binding domains binding to one or several, for example one to three MHC I - HPV 16 E6 T-cell epitope complexes, expressed on epithelial tumor cells, for the targeting of squamous tumors induced upon HPV infection. Examples of suitable target HPV 16 E6 T-cell epitopes are peptides FQDPQERPR, TTLEQQYNK, ISEYRHYCYS and GTTLEQQYNK binding to HLA A1, KISEYRHYC and YCYSIYGTTL binding to HLA A2, LLRREVYDF and IVYRDGNPY binding to HLA A3, TTLEQQYNK binding to HLA A11, CYSLYGTTL, KLPQLCTEL, HYCYSLYGT, LYGTTLEQQY, EVYDFAFRDL AND VYDFAFRDLC binding to HLA A24, 29-TIHDIILECV-38 binding to HLA A*0201. Equally suitable are HPV 16 E7 T-cell epitopes such as 86-TLGIVCPI-93, 82-LLMGTLGIV-90, 85-GTLGIVCPI-93 and 86-TLGIVCPIC-94 binding to HLA A*0201, HPV 18 E6 T-cell epitopes and HPV 18 E7 T-cell epitopes, binding to HLA A1, A2, A3, A11 or A24. Yet additional examples of T-cell epitopes related to HPV infected cells are HPV E7 derived peptides 1-MHGDTPTLHEYD-12, 48-DRAHYNIVTFCCKCD-62 and 62-DSTLRLCVQSTHVD-75 binding to HLA DR, 7-TLHEYMLDL-15, 11-YMLDLQPETT-20, 11-YMLDLQPET-19 and 12-MLDLQPETT-20 binding to HLA A*201, 16-QPETTDLYCY-25, 44-QAEPDRAHY-52 and 46-EPDRAHYNIV-55 binding to HLA B18, 35-EDEIDGPAGQAEPDRA-50 binding to HLA DQ2, 43-GQAEPDRAHYNIVTFCCKCDSTLRLCVQSTHVDIR-77 binding to HLA DR3, 50-AHYNIVTFCCKCD-62 binding to HLA DR15, 58-CCKCDSTLRLC-68 binding to HLA DR17 and 61-CDSTLRLCVQSTHVDIRTLE-80 binding to HLA-DRB1*0901. Examples of alternative keratinocyte markers to which low-affinity binding domains in binding molecules of the invention can bind are human gene encoding keratinocyte proline-rich protein, glycoprotein-80 and 174H.64.

For the treatment of health problems related to exposure to agents used for acts of bioterrorism and biological warfare, multi-specific binding molecules are designed as part of the invention. For example, binding molecules of the invention comprise different binding domains with specificity for two or more agents used for biological warfare. Examples are the combination of one or more binding domains specific for anthrax, combined with one or more binding domains specific for botulinum neurotoxin. In this way, a few multi-specific binding molecules are designed which are useful for the prophylaxis or treatment of all the commonly acknowledged biological warfare threats, and which can be stock piled. This provides the benefits of being prepared for attacks by the common agents, by producing, purifying and stock-piling only a few different multi-specific proteinaceous molecules of the invention.

It is one of the advantages of the current invention that immune escape mechanisms of pathogens are effectively counteracted upon use of the binding molecules of the invention. For example, the binding molecules according to the invention are multi-specific, in a monovalent or multivalent manner, for pathogen associated molecular patterns (PAMPs). In this way, the probability for occurrence of immune escape is strongly reduced. Proteinaceous molecules of the invention will only then not be able to bind to the targeted pathogen anymore in the unlikely situation when all binding sites on the PAMP(s) are mutated simultaneously in a way that binding affinity is lost completely. Thus, the proteinaceous molecules of the invention can still exert at least partially a desired therapeutic effect as long as at least one binding site on a PAMP remains unaltered while the other, or one or more of the other, or even all other binding sites are mutated on the pathogen surface.

One example of proteinaceous molecules of the invention are molecules comprising multiple different Vh domains, in monovalent or multivalent form, specific for multiple isotypes or serotypes of the same virus. By doing so, the area of therapeutic use of the binding molecules is expanded, covering a broader range of viral subtypes. An examples that is provided are proteinaceous molecules of the invention comprising binding domains specific for conserved epitopes in the F subdomain of influenza A virus hemagglutinin glycoprotein. Such proteinaceous molecules of the invention are of particular use in the treatment of infection with influenza virus of any of the known A subtypes.

### Example 2: Selection of antibody fragments

Multi-specific proteinaceous molecules of the invention are built from any antigen binding domain, such as, but not limited to, antibodies, alpha-helices and T-cell receptors. Antibody Vh fragments specific for pathogens or for pathogen associated surface antigens are derived from hybridoma cells producing mouse, rat, rabbit, llama or human antibodies. Antibody fragments can also be obtained after immunization of animals with pathogen (cells) or (partly) purified pathogen antigen. Alternatively, antibody fragments of human, mouse, rat or llama origin can be obtained from antibody phage, yeast, lymphocyte or ribosome display libraries. Such antibody libraries (scFv, Fab, Vh or Vhh) may be constructed from non-immunized species as well as immunized species.

### 2.1: Selection of human antibody fragments specific for pathogen antigens or cell-surface expressed pathogen-associated antigens.

To obtain human antibody fragments specific for a surface molecule on a pathogen or for a pathogen associated antigen expressed at the surface of an invaded cell, a Human antibody Fab, VHCH or Vh phage display library will be used for selections essentially as described by Chames *et al.* Human Fab phages (10¹³ colony forming units) are first pre-incubated for 1 h at room temperature in PBS containing 2% non-fat dry milk (PBSM). In parallel, 200 µl Streptavidin-coated beads (Dynal) are equilibrated for 1 h in PBSM. For subsequent rounds, 100 µl beads are used. Equilibrated beads are added, and the mixture is incubated for 15 minutes under rotation. Beads are drawn to the side of the tube using magnetic force. To the depleted phage fraction, subsequently decreasing amounts of biotinylated target antigen are added and incubated for 1 h at room temperature, with continuous rotation. Equilibrated streptavidin-coated beads are added, and the mixture incubated for 15 minutes under rotation. Phages are selected by magnetic force. Non-bound phages will be removed by 5 washing steps with PBSM, 5 steps with PBS containing 0.1% Tween, and 5 steps with PBS. Phages are eluted from the beads by 10 minutes incubation with 500 µl freshly prepared tri-ethylamine (100 mM). The pH of the solution is then neutralized by the addition of 500 µl 1 M Tris (pH 7.5). The eluted phages are incubated with logarithmic growing *E. coli* TG1 cells (OD₆₀₀ₙₘ of 0.5) for 30 minutes at 37°C. Bacteria are grown overnight on 2x TYAG plates. Next day, colonies are harvested, and a 10 µl inoculum is used in 50 ml 2x TYAG. Cells are grown until an OD₆₀₀ₙₘ of 0.5, and 5 ml of this suspension is infected with M13k07 helper phage (5x10¹¹ colony forming units). After 30 minutes incubation at 37°C, the cells are centrifuged, resuspended in 25 ml 2x TYAK, and grown overnight at 30°C. Phages are collected from the culture supernatant as described previously, and used for the next round panning. After two, three or four selection rounds enrichment of specific binders is obtained, and individual clones are analyzed for binding to specific influenza virus by ELISA.

### Example 3: Production of multi-specific proteins comprising camelised single domain Vh domains.

### 3.1: design of genes for production of multi-specific Vh proteins.

Human antibody germline gene VH3 demonstrates high homology to llama single domains VHH. Exchange of amino-acids 44, 45 and 47 in the human VH3 genes by amino-acids present in llama VHH at these positions has shown to enhance stability and expression of the human VH3 genes [Riechmann, Muyldermans, 199]. For expression and stability many of the selected human Vh might benefit from the exchange of amino-acids 44, 45 and 47 by llama VHH amino-acids, a process called camelization. A gene comprising at least two distinct human Vh domains binding to at least two distinct pathogen epitopes or pathogen-associated cell surface epitopes of invaded cells will be compiled such that upon expression it would comprise six Vh domains. To this end a gene will be designed comprising the pelB secretion signal, which will be operatively linked to six codon-optimized, camelized Vh domains with linkers ((Gly₄Ser)ₙ, (GSTSGS)ₙ, GSTSGSGKPGSGEGSTKG, EFAKTTAPSVYPLAPVLESSGSG or any other linker that provides flexibility for protein folding, or, EPKSCDKTHT (IgG1), ELKTPLGDTTHT (IgG3), or ESKYGPP (IgG4)) between each Vh domain. This gene will for example be synthesized by Geneart (Regensburg, Germany) and cloned into the pStaby 1.2 vector (Delphi genetics, Belgium) for expression in *E. coli.*

### Example 4: production and purification of Hexameric Vh protein.

For expression of multi-specific Vh proteins the pStaby-multispecific-protein vectors will be introduced via electroporation into SE1 bacteria. Positive clones will be grown in the presence of 2% glucose at 25-30°C until OD₆₀₀ = 0.8. Bacterial TYAG medium will then be replaced with TY medium containing 0,1-1 mM IPTG to induce expression. After overnight culture at 25-30°C bacteria and medium will be harvested. The periplasm fraction will be collected after incubation of bacteria with PBS/EDTA/NaCl for 30 minutes on ice. Protein expression will then be analyzed by SDS-PAGE.

Multi-specific Vh proteins will be isolated from media and bacteria using Ni-affinity purification. To this end medium will be incubated with Ni-coupled Sepharose-beads and incubated overnight while stirring gently. To obtain intracellular proteins bacteria will be lysed and cellular debris removed by centrifugation. After overnight dialysis with PBS multi-specific Vh proteins will be purified with Ni-Sepharose. Purity of the multi-specific Vh proteins will be analyzed by SDS-PAGE and protein concentration determined by BCA protein assay (Pierce).

### Example 5: Construction of multi-specific genes to improve circulation.

The pharmacokinetic properties of therapeutic proteins, e.g. their distribution, metabolism and excretion are dependent on factors such as shape, charge and size. Most small plasma molecules (MW< 50-60 kDa) possess very short half-life, whereas larger plasma proteins such as human serum albumin (HSA) and immunoglobulins (Ig) have very long half-lives (19 days for HSA, 1-4 weeks for Ig). Indeed, addition of IgG-Fc or Human serum albumin has shown to extend circulation time when linked to therapeutic proteins. In addition the coupling of IgG-Fc to the multi-specific proteins will allow recruitment of immune cells to the site of infected cells allowing immune specific responses against the colonized tissue.

### 5.1: construction of multi-specific proteins with IgG1-Fc and human serum albumin.

The multi-specific construct will be linked to the IgG1-Fc region or to human serum albumin, codon optimised for expression in eukaryotic cells and cloned into the pcDNA-3.1+ vector (Geneart, Regensburg, Germany).

### Example 6: Isolation of a binding domain for a HCV epitope (KLVALGINAV) in the context of HLA A02.01 from a FAB phage display library.

### 6.1: Selection of human antibody fragments specific for pathogen antigens presented by HLA-A02.01.

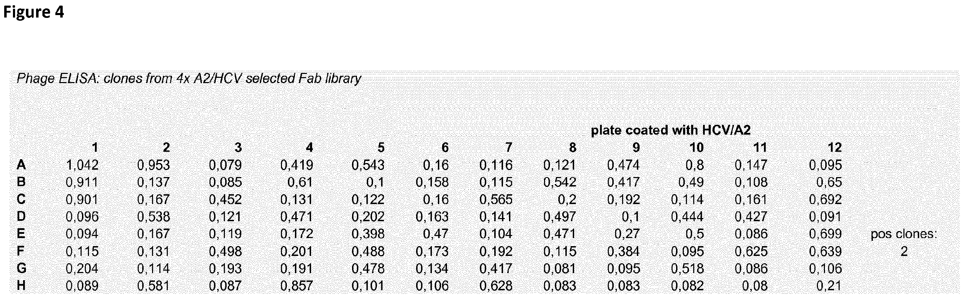

For selection of human antibody fragments specific for the HCV epitope KLVALGINAV presented by HLA-A02.01 on the surface of infected cells, essentially the protocol as outlined in Example 2 was conducted (See above). Thus, to obtain human antibody fragments specific for a HCV epitope (KLVALGINAV) presented by HLA-A02.01 at the surface of an infected cell, a human antibody Fab phage display library was used for selections. For the first selection round, human Fab phages (10¹³ colony forming units) were first pre-incubated for 1 h at room temperature in PBS containing 2% non-fat dry milk (PBSM). In parallel, 200 µl Streptavidin-coated beads (Dynal) were equilibrated for 1 h in PBSM. For a subsequent second, third and fourth selection round, 100 µl beads were used. Equilibrated beads were added, and the mixture was incubated for 15 minutes under rotation. Beads were drawn to the side of the tube using magnetic force. To the depleted phage fraction, subsequently decreasing amounts of biotinylated target antigen were added and incubated for 1 h at room temperature, with continuous rotation. Equilibrated Streptavidin-coated beads were added, and the mixture incubated for 15 minutes under rotation. Phages were selected by magnetic force. Non-bound phages were removed by 5 washing steps with PBSM, 5 steps with PBS containing 0.1% Tween, and 5 steps with PBS. Phages were eluted from the beads by 10 minutes incubation with 500 µl freshly prepared tri-ethylamine (100 mM). The pH of the solution was then neutralized by the addition of 500 µl 1 M Tris (pH 7.5). The eluted phages were incubated with logarithmic growing *E. coli* TG1 cells (OD₆₀₀ₙₘ of 0.5) for 30 minutes at 37°C. Bacteria were grown overnight on 2x TYAG plates. Next day, colonies were harvested, and a 10 µl inoculum was used in 50 ml 2x TYAG. Cells are grown until an OD₆₀₀ₙₘ of 0.5, and 5 ml of this suspension was infected with M13k07 helper phage (5x10¹¹ colony forming units). After 30 minutes incubation at 37°C, the cells were centrifuged, resuspended in 25 ml 2x TYAK, and grown overnight at 30°C. Phages were collected from the culture supernatant as described previously, and used for the next round panning. After four selection rounds individual clones were analyzed for specific binding to the complex of HLA-A02.01 and the HCV peptide epitope KLVALGINAV by ELISA (Figure 4). The ELISA results revealed that after the fourth selection round two antibody Fab molecules were selected, enriched for specific binding to the complex of HLA-A02.01 and the HCV peptide epitope KLVALGINAV.

### Expample 7: Production of a mono-specific protein comprising six camelised single domain Vh domains.

### 7.1: Design of genes for production of a hexameric mono-specific Vh protein specific for the complex of HLA-A02.01 and the HCV peptide epitope KLVALGINAV.

A hexameric monospecific protein comprising six Vh domains specific for the complex of HLA-A02.01 and the HCV peptide epitope KLVALGINAV and obtained with the selection procedure outlined in Example 6.1, above, will be constructed essentially as outlined in Example 3.1 (See above).

### 7.2: Production and purification of hexameric monospecific Vh protein.

Production and purification of the hexameric mono-specific Vh protein specific for the complex of HLA-A02.01 and the HCV peptide epitope KLVALGINAV as will be obtained according to Example 7.1, will essentially be performed according to the protocol provided in Example 4.

### Example 8: Apoptosis inducing activity of the hexameric mono-specific Vh protein specific for the complex of HLA-A02.01 and the HCV peptide epitope KLVALGINAV towards cells presenting this complex

The apoptosis inducing capacity of purified hexameric monospecific binding molecules specific for the complex of HLA-A02.01 and the HCV peptide epitope KLVALGINAV towards mammalian cells presenting this complex are demonstrated with HLA-A02.01 expressing HCV infected cells. To this end, HCV-infected mammalian cells are either exposed to the hexameric monospecific binding molecules specific for the complex of HLA-A02.01 and the HCV peptide epitope KLVALGINAV, or to a negative control (e.g. a non-binding hexameric Vh molecule and/or assay buffer only). In addition, as a negative control, non-infected cells are exposed to the hexameric monospecific binding molecules. Apoptosis inducing activity of the hexameric monospecific binding molecules towards HCV-infected cells is quantified by analyzing (the) amount(s) of apoptotic marker molecule(s) exposed or secreted by these infected cells (e.g. caspase) exposed to the hexameric monospecific binding molecules specific for the complex of HLA-A02.01 and the HCV peptide epitope KLVALGINAV, compared to (the) amount(s) of apoptotic marker molecules exposed or secreted by the controls.

### Figure legends

### Figure 1. Exemplified topologies of binding molecules comprising one or more copies each of two or more different binding domains each binding to a different binding site, according to the invention, and in one embodiment comprising effector moieties as part of the invention.

1. Topologies of binding molecule comprising two different binding domains 'D1' and 'D2', and divalent for a binding site 1 and monovalent for a binding site 2.
2. Binding molecule comprising two different binding domains and monovalent for a binding site 1 and multivalent for a binding site 2 (multi-valency is for example 3-6). Shown are two examples of many possible single-chain polypeptides according to the invention. All possible permutations regarding the position of the single binding domain and the multiple copies of the second binding domain are also part of the invention, and are visualized by the ensemble of different domains and number of domains between accolades.
3. Binding molecule comprising two different binding domains each binding to a different binding site and with two to six copies of a first binding domain and with two to six copies of a second binding domain, providing multi-valency for both binding sites. As an example, a binding molecule is shown in which binding domains binding to the same binding site are linked in consecutive order. All possible domain topologies obtained by permutations regarding domain positions in the single chain binding molecule of all binding domains of both kinds, are also part of the invention.
4. Binding molecule comprising three, four, five or six different binding domains, thus binding to three, four, five or six different binding sites, respectively, and monovalent or multivalent for a binding site 1, monovalent or multivalent for a binding site 2, etc. (the valencies for the three to six different binding sites are for example one to six). As an example, four binding molecules are shown in which one to six clustered identical binding domains are linked in consecutive order, with three, four, five and six different binding domains in the binding molecules, respectively. All possible domain topologies obtainable by permutations regarding domain positions in the single chain binding molecule, of all one to six copies of the three to six different binding domains, are also part of the invention.
5. Binding molecule comprising two different binding domains each binding to a separate binding site and with one binding domain monovalent or multivalent for a binding site 1 and the second binding domain monovalent or multivalent for a binding site 2 (both valencies are for example 1-6), and with one or more effector moieties (covalently) bound to the binding molecule. As an example, a binding molecule is shown in which the two sets of one to six binding domains are linked in consecutive order, with the effector moiety covalently linked to the C-terminus of the binding molecule. All possible domain topologies obtainable by permutations regarding each domain position in the single chain binding molecule are also part of the invention.
6. Similar to 5., now with three to six different binding domains, for each of which one to six copies of the unique binding domains are part of the binding molecule.

### Figure 2. Exemplified topologies of multi-specific binding molecules comprising two or more different binding domains each binding to a different binding site, and linked to an Fc monomer, which is in one embodiment provided as a single chain molecule and in a second embodiment as a two-chain Fc fragment comprising two Fc monomers with arms each comprising two or multiple binding domains, according to the invention.

1. Binding molecule comprising one or multiple, preferably one to six copies each of two different binding domains, thus mono-specific or multi-specific for a binding site *a* and mono-specific or multi-specific for a binding site *b*, and comprising an Fc monomer. Shown is an example of a single-chain molecule according to the invention with n binding domains specific for binding site *a* and m binding domains specific for binding site *b*. All possible domain topologies regarding the position of the individual binding domains specific for binding site *a* or *b* are also part of the invention. It is also part of the invention that a third, fourth, etc. type of binding domain (preferably one to six copies of each different binding domain) are incorporated in binding molecules with specificity for a third, fourth, etc. unique binding site *c*, *d*, etc.
2. Two-chain binding molecule formed upon dimerization through binding interactions between two Fc monomers of a binding molecule comprising multiple, preferably two to six identical binding domains, thus mono-specific for a binding site *a*, and comprising an Fc monomer, forming an Fc fragment with two identical arms. Alternatively, two Fc monomers comprising arms with different binding domains and/or with different numbers of copies of identical binding domains, are dimerized, resulting in two-chain hetero-dimeric binding molecules of the invention comprising Fc fragments with bi-specific arms.
3. Two-chain binding molecule formed upon dimerization through binding interactions between Fc monomers as outlined in **1**., forming an Fc fragment with two identical arms. Alternatively, two Fc monomers comprising different arms with different binding domains and/or with different domain topologies, are dimerized, resulting in two-chain hetero-dimeric binding molecules of the invention comprising Fc fragments with bi-specific arms, with the number of copies n, m, o and p of binding domains each being two to six.

Examples of multiple different binding sites targeted in a monovalent or multivalent manner by different binding domains are given in the specification and in Example 1. An effector moiety can be part of any of the outlined proteinaceous molecules, as detailed in the specification.

### Figure 3. Cartoon displaying examples of preferred domain topologies.

Examples are provided of possible combinations of V_{H} domains and distinct linker sequences for the construction of multi-domain proteins that are mono-specific or multi-specific. In *a-h.* various examples are provided of proteinaceous molecules of the invention, comprising two or three different binding domains, and comprising one, two, three or four copies of the various binding domains, each, all linked with two or three different linkers (See also Figure 1, example 1.-4. and Figure 2 for additional preferred domain topologies of the invention). In *i.* and *k*., the exemplified preferred proteinaceous molecules of the invention further comprise an effector moiety linked to the single chain polypeptide comprising different binding domains (Additional preferred proteinaceous molecules of the invention comprising at least one effector moiety are provided in examples 5. and 6., in Figure 1). In *j*. and *k*., the exemplified preferred proteinaceous molecules of the invention further comprise an Fc monomer linked to the different binding domains (See also Figure 2). In 1. an example is provided of a preferred mono-specific proteinaceous molecule of the invention.

### Figure 4. Elisa results of 4th selection

The extinction results at OD450 showing the binding of Fab fragments to HCV/A2 antigens.

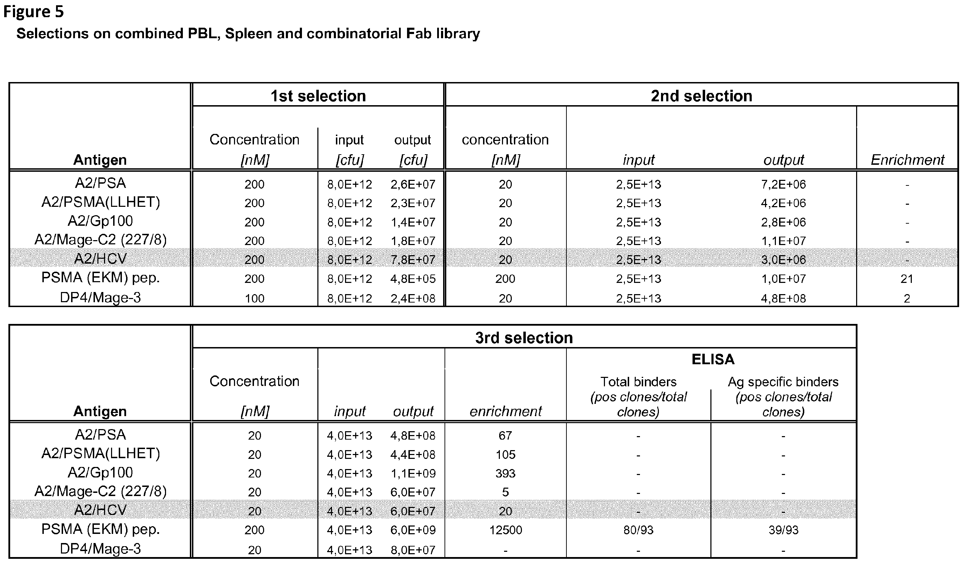

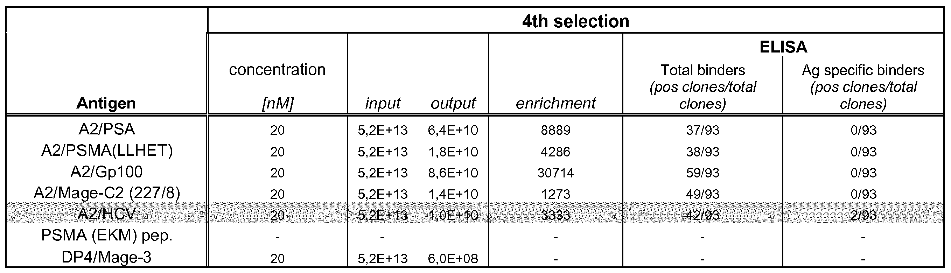

### Figure 5. Selections on combined PBL, Spleen and combinatorial Fab library.

The results of the four selection rounds using the complex of HLA-A02.01 and the HCV peptide epitope KLVALGINAV and a combined PBL, spleen and combinatorial Fab library are separately displayed for each selection round. Finally, after the fourth round, two specific clones were obtained.

### References

- Ridgway, J.B., Presta, L.G. and Carter, P., 'Knobs-into-holes' engineering of antibody CH3 domains for heavy chain heterodimerization, Protein Engineering 1996, 9(7), 617-621.
- Van den Eynde, B.J., van der Bruggen, P., T cell-defined tumor antigens. Curr Opin Immunol 1997, 9, 684-93.
- Houghton, A.N., Gold, J.S., Blachere, N.E., Immunity against cancer: lessons learned from melanoma. Curr Opin Immunol 2001, 13, 134-40.
- van der Bruggen, P., Zhang, Y., Chaux, P., Stroobant, V., Panichelli, C., Schultz, E.S., Chapiro, J., Van den Eynde, B.J., Brasseur, F., Boon, T., Tumor-specific shared antigenic peptides recognized by human T cells. Immunol Rev 2002, 188, 51-64.
- Parmiani, G., De Filippo, A., Novellino, L., Castelli, C., Unique human tumor antigens: immunobiology and use in clinical trials. J Immunol 2007, 178, 1975-9.

## Claims

1. A proteinaceous molecule comprising at least two specific binding domains separated by at least one linker wherein said proteinaceous molecule comprises a single polypeptide chain wherein said binding domains specifically recognize binding sites present on or associated with at least one pathogen or a cell infected with a pathogen.

2. A proteinaceous molecule according to claim 1, comprising at least two specific binding domains for different binding sites separated from each other by at least one linker.

3. A proteinaceous molecule according to claim 1, comprising at least three specific binding domains for different binding sites separated from each other by at least one linker.

4. A proteinaceous molecule according to claim 1 or 2, wherein at least one such a binding domain is a Vh domain.

5. A proteinaceous molecule according to any one of claims 1-3, further comprising an effector moiety.

6. A proteinaceous molecule according to any one of claims 1-4 comprising at least two Vh domains.

7. A proteinaceous molecule according to any one of claims 1-4, comprising at least two Vh domains specific for different binding sites and an Fc monomer.

8. A dimeric proteinaceous molecule, comprising two proteinaceous molecules according to claim 6 dimerized through two Fc monomers.

9. A hetero-dimeric molecule according to claim 7, comprising two different proteinaceous molecules according to claim 6.

10. A proteinaceous molecule according to any one of claims 1-8 for use in the treatment of an infectious disease.

11. A pharmaceutical formulation comprising a proteinaceous molecule according to any one of claims 1-9 and suitable excipients.

12. A nucleic acid molecule encoding a proteinaceous molecule according to any one of claims 1-8.

13. A vector comprising a nucleic acid molecule according to claim 11.

14. A cell comprising a nucleic acid molecule according to claim 11, preferably integrated in its genome and/or a vector according to claim 12.

15. A method for producing a proteinaceous molecule according to any one of claims 1-8, comprising culturing a cell according to claim 13, allowing for expression of the proteinaceous molecule and separating the proteinaceous molecule from the culture.

16. A proteinaceous molecule comprising at least four specific binding domains for the same binding site separated by at least one linker wherein said proteinaceous molecule comprises a single polypeptide chain, for use in the treatment of an infectious disease.

17. A proteinaceous molecule according to Figure 1, Figure 2 or Figure 3.
